Europäisches Patentamt

European Patent Office

Office européen des brevets

(19)

(11) Veröffentlichungsnummer: **0 454 877 A1**

## (12) EUROPÄISCHE PATENTANMELDUNG

(21) Anmeldenummer: 90108190.1

(22) Anmeldetag: 28.04.90

(51) Int. Cl.⁵: **A61N 1/16**

(43) Veröffentlichungstag der Anmeldung:
**06.11.91 Patentblatt 91/45**

(84) Benannte Vertragsstaaten:
**AT BE CH DE DK ES FR GB GR IT LI LU NL SE**

(71) Anmelder: **Kehlbeck, Heinrich**
**Bahnhofstrasse 2**
**W-2812 Hoya(DE)**

(72) Erfinder: **Kehlbeck, Heinrich**
**Bahnhofstrasse 2**
**W-2812 Hoya(DE)**

(74) Vertreter: **Rücker, Wolfgang, Dipl.-Chem.**
**Patentanwalt**
**Bergiusstrasse 2b**
**W-3000 Hannover 51(DE)**

(54) Verfahren und Vorrichtung zur Übertragung von Photonenenergie bzw. Schwingungsenergie auf eine Flüssigkeit.

(57) Beschrieben wird ein Verfahren und eine Vorrichtung zur Übertragung von Photonenenergie bzw. Schwingungsenergie von Stoffen organischer und anorganischer Art auf eine Flüssigkeit, insbesondere Wasser, wobei die energieliefernden Stoffe in ein Röhrchen oder eine Dose aus Glas oder Aluminium, vorzugsweise aus Quarzglas, eingeschlossen werden und dieses in einer Flüssigkeit bewegt wird. In der Dose (1) liegt eine präparierte Platte (5), die mit Halbleitern (8,9) beschichtet ist,z.B. mit Germanium. Zusätzlich können Substanzen (11) auf die Platte geschichtet sein. Die Flüssigkeit ist in einem Becher (12) enthalten, den man auf die Platte bzw. auf die Dose (1) stellt.

EP 0 454 877 A1

Es ist bekannt, Medikamente elektrisch zu messen, ähnlich wie beim EKG oder EEG. Bei der Medikamentenmessung oder -testung wird dem Patienten eine Elektrode in die Hand oder unter den Fuß gegeben und mit einer 2. Elektrode sein Körper auf bestimmte Linien oder Punkte unter Anlegung einer Spannung abgetastet. Bei dieser Meßmethode erhält man Widerstandskurven, die sich graphisch darstellen lassen, und man kann so angeblich feststellen, ob Medikamente für den betreffenden Organismus heilend wirken oder nicht. Es ist also möglich, mit diesem Verfahren patientenspezifische Heilmittel mit hoher Sicherheit auszuwählen (Biologische Medizin, Heft 4, August 1987, Seite 512 ff).

Ähnliche Effekte wie bei der medizinischen Testung oder Messung treten auch bei Pflanzen auf. Man vermutete ultraschwache Lichtstrahlen bei lebenden Proben, wie Gurken- und Kartoffelkeimen u.a. Eine solche Strahlung ist an das Leben gebunden und verschwindet bei Abtötung der Keime nach vorhergehender Erhöhung. Die Emission dieser Biophotonen lebender Keime ändert sich bei Annäherung potenzierter Substanzen, ohne daß eine chemische Einflußmöglichkeit vorliegt. (Ruth, Bernhard, Experimental Investigations on Ultraweak Photon Emission. Enthalten in Popp, F.A. Becker, G. König, H.L., Peschka, W. (Ed.): Electro-magnetic Bio-Information. Urban & Schwarzenberg, München 1979). (Höllischer, E. und Mehlhardt, W., Untersuchungen zur Objektivierbarkeit des EAV-Medikamententests durch Messung der Emission von Biophotonen - eine vorläufige Mitteilung, Ärztezeitschrift f. Naturheilverf. 22,6, 316 - 317 (1981).

Andererseits ist es nun bekannt, daß Pflanzen Licht leiten und daß entlang der Meridiane der menschliche Körper Lichtleiterfunktion besitzt. Es wurde entdeckt, daß Gerstenstengel Licht über eine Entfernung von 4 - 5cm leiten können. Das Licht, das diese Strecke zurückgelegt hatte, wurde visuell und mit Hilfe einer Photomultipliziervorrichtung am unbeleuchteten Stengelende registriert. Es wurde ferner beobachtet, daß paradoxerweise die Pflanze beträchtlich gebogen war, nachdem das Licht durch sie hindurchgegangen war. Es ist möglich, daß der innere Lichtverbrauch dem Organismus hilft, auf Veränderungen von Umweltbedingungen zu reagieren und die Funktionen verschiedener Körperteile entsprechend zu regulieren. Zum Beispiel verfügen Pflanzen über kein Äquivalent zum Nervensystem höherer Spezies. So könnte es sein, daß manchmal die Lichtleiterkanäle diese Funktion übernehmen. Im menschlichen Organismus ist das innere Lichtsystem wahrscheinlich eines der ältesten Regulierungseinrichtungen, die wir von den frühesten Stufen der Evolution geerbt haben, aus den Zeiten, als das Nervensystem lebender Organismen noch unterentwickelt oder auch noch gar nicht vorhanden war (raum & zeit 35/88, Seite 17 und 18).

Erfindungsgemäß besteht nun die Aufgabe darin, diese Frequenzen bzw. geringen Ströme, durch die die Photonen in biochemischen Vorgängen, insbesondere von pflanzlichen, tierischen und mineralischen Stoffen, auftreten, zu nutzen, um für Erkrankungen vorteilhafte, der Therapie dienliche Erkenntnisse zu erhalten.

Gelöst wird diese Aufgabe dadurch, daß diese pflanzlichen, tierischen oder mineralischen Stoffe in ein Röhrchen eingefüllt werden, und das Röhrchen mit einer Flüssigkeit in Berührung gebracht wird.

Das Röhrchen ist vorzugsweise ein Quarzglasröhrchen, das wenigstens einendig verschlossen ist und in welchem der zu prüfende pflanzliche, tierische oder mineralische Stoff enthalten ist. Die Flüssigkeit, in die das Röhrchen mit dem Stoff eingetaucht wird, ist Wasser oder auch jedes andere Getränk, z.B. Mineralwasser.

Die Verweilzeit für das Röhrchen im Wasser ist je nach Stoff unterschiedlich, beträgt aber im allgemeinen 1 - 2 Minuten. Mißt man nun an jenen oben aufgezeigten Linien des menschlichen Körpers, beispielsweise an den Meridianen, so registriert man Ströme äußerst geringer Stärke und bestimmter Frequenzen, deren Intensität Aufschluß über die Verträglichkeit oder Effektivität der verabreichten Präparate geben, wenn der Patient die so behandelte Flüssigkeit zu sich nimmt. Der Stoff, dessen Energie auf die Flüssigkeit übertragen wird, kann, wie aufgeführt, organischer oder anorganischer Art sein. So kann das Röhrchen beispielsweise enthalten: Grundelemente, wie Halbleiter, z.B. Germanium, Silicium oder pflanzliche, d.h. organische Stoffe, wie Extrakte oder Teile von Pflanzen, beispielsweise Weißdorn oder von Knoblauch, Vitamine usw. Diese Stoffe werden in das als Rührstab fungierende Röhrchen eingebracht, und dann wird der Rührstab in einem Behälter mit dem Wasser bewegt. Der Rührstab sollte vorzugsweise eine flache Form haben und aus Quarzglas bestehen, also keinen kreisrunden Querschnitt, sondern eher einen flach-ovalen Querschnitt besitzen. In manchen Fällen ist es auch möglich, einen solchen Rührstab statt aus Quarzglas aus reinem Aluminium zu fertigen und damit die Flüssigkeit zu rühren.

Das Wasser kann auch einen Anteil Äthylalkohol enthalten. Wenn man z.B. eine neutrale Flüssigkeit, wie Aqua bidest oder wie Kaffee, Tee, Milch, Mineralwasser usw., mit dem oben beschriebenen Rührstab, in dem der oben erwähnte Stoff mit seiner ihm eigenen Energie sich befindet, rührt, dann wird diese innerhalb kurzer Zeit auf die Flüssigkeit übertragen, so daß auch insofern dem Patienten oder Probanten diese Energie übertragen wird und er in bestimmter Weise reagiert. Wenn einem Menschen z.B. bei Herzbeschwerden eine Flüssigkeit verabreicht wird, beispielsweise Wasser, das mit einem Rührstab gerührt worden ist, der Crategutt forte (Weißdornpräparat) enthält, dann wird die Energie dieser Flüssigkeit und damit die des Präparates von der Mundschleimhaut und Magen/Darm aufgenommen, was mit entsprechenden Meßgeräten feststellbar ist. Bei Stoffen oder Präparaten, die aufgrund ihres Geruchs lästig sind oder abgelehnt werden, wie beispielsweise Knoblauch, kann dieser sogar in konzentrierter Form in einen solchen Rührstab eingebracht und mit Wasser behandelt werden, und man erzielt Wirkungen, die dem Genuß der Pflanze selbst entsprechen, ohne daß lästige Nebenwirkungen, wie Geruch, sich bilden.

Diese erfindungsgemäße Verarbeitung bzw. Applikation des energetischen Inhalts von Stoffen der oben beschriebenen Art läßt sich auch auf Pflanzen anwenden, wenn man beispielsweise das Gießwasser entsprechend behandelt oder den Stab in den Blumentopf, in die Nähe der Wurzel steckt.

So wurde z.B. bei einer breitblättrigen Pflanze (Gummibaumart) mit einem Durchmesser von 60 cm kein Lebenszeichen mehr registriert, und die Blätter wiesen großflächige braune Stellen auf, in denen sich das "Sterben" der Pflanze anzeigte. Es war durch Messen der biologischen Energie keine Resonanz festzustellen. Ein mit einem Germanium-Präparat beschickter Rührstab wurde in die nasse Erde gesteckt und für Sekunden darin belassen. Bereits nach 3 Tagen entwickelte die Pflanzenmitte neue Triebe. Daraus läßt sich schließen, daß diese geringen Lichtenergien biochemische Vorgänge in pflanzlichen und tierischen Zellen steuern bzw. beeinflussen können.

Oben ist die Lösung der Aufgabe damit beschrieben worden, daß man ein Röhrchen mit den genannten Stoffen gefüllt, also einendig verschlossen, in einer Flüssigkeit bewegt, d. h. und die Flüssigkeit damit rührt.

In Verfolgung des erfinderischen Gedankens kann die Erfindung auch dadurch verwirklicht werden, daß man die beteiligten Mittel und Verfahrensstufen umkehrt.

Dies geschieht dadurch, daß man eine Dose mit einem Deckel aus Glas - ähnlich einer Petrischale - einsetzt, auf deren Boden im Inneren zur Erzeugung einer Photonenstrahlung ein Träger eingelegt ist, der in seiner Gestalt den Innenabmessungen der Petrischale entspricht und aus Zellstoff, in der Art einer Pappe oder aus einem Kunststoff - wie Zelluloid -, besteht, und der mit einem Halbleiter beschichtet ist, z.B. in Form eines Lackes. Vorzugsweise ist der Träger doppelt vorhanden, sodaß die beiden nach oben und unten weisenden Oberflächen beschichtet sind- während die einander zugekehrten Oberflächen nicht beschichtet sind, dadurch ergeben sich unterschiedliche Polaritäten.

Die beschichteten Seiten sind dabei positiv und die nicht beschichteten Seiten negativ. Zwischen beiden Schichten kann eine Aluminium-Folie liegen und das ganze Schichtstoffgebilde durch eine Abdeckplatte bedeckt sein. Zur Verstärkung der Photonenstrahlung kann auf den Schichtstoff Substanz geschichtet werden, die eine Verstärkung der Photonenstrahlung bewirkt, analog der Füllung in dem oben beschriebenen Glasröhrchen, dessen Querschnitt rund oder oval oder ähnlich sein kann. Auf den Schichtstoff werden Substanzen geschichtet, die Heilpflanzen, Arzneimittel, Teemischungen und dergleichen sein können. Diese verstärkend wirkenden Stoffe oder Substanzen können auch in wässriger oder alkoholischer Form und selbstverständlich fein zerteilt als Pulver oder in natürlicher Form vorliegen.

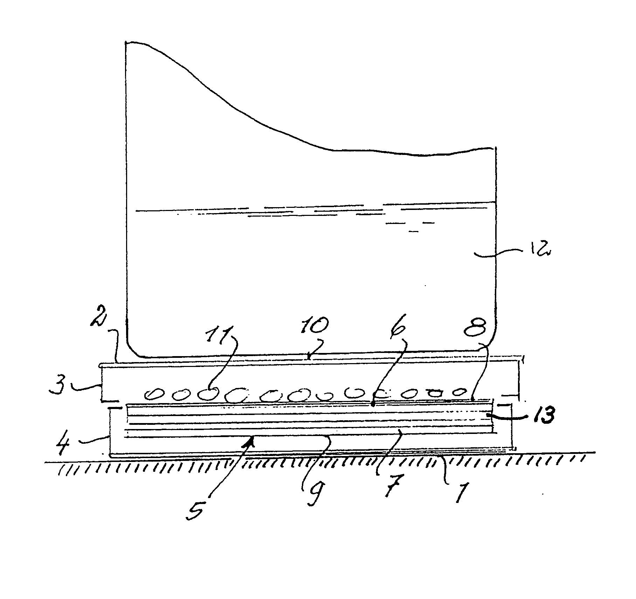

Auf die so getroffene Anordnung wird der Schalendeckel aufgesetzt und wie in der ersten Ausführung das Gefäß mit der Flüssigkeit auf den Dosendeckel gesetzt. Es hat sich gezeigt, daß die Wirkung der Dose mit der darin befindlichen Halbleiter beschichteten Platte eine erhebliche Verstärkung verursacht. Diese Verstärkung läßt sich messen. Dabei liegt die Dose flach, wie in der Zeichnung dargestellt. Steht sie hochkant, erhält man keine Werte oder nur ganz schwache.

Der Durchmesser des Gefäßes entspricht etwa dem Durchmesser der Petrischale. Natürlich muß die Schale nicht unbedingt eine Petrischale sein, jedoch ist die flächige Petrischale relativ großen Durchmessers und wegen des weit übergreifenden Deckels für den erfindungsgemäßen Zweck gut geeignet. Die Schale oder die Dose kann auch aus Kunststoff bestehen. Jedenfalls sollte sie vorzugsweise aus einem Viskolyt bestehen.

Die Herrichtung des Germanium-Lackes zur Beschichtung analog der Dose erfolgt unter Verwendung von Germaniumoxyd oder einer anderen geeigneten Germanium-Verbindung und nach allgemeinen Regeln der Lackherstellung. Anstelle der Germanium-Substanz kann auch ein anderer Halbleiter eingesetzt werden, beispielsweise fein zerteiltes Siliciumoxyd oder feinteiliger Quarzsand. Die Verbindung mit der Platte muß nicht mit Lack erfolgen; sie kann in anderer Form zum Haften gebracht werden, z.B. durch Tauchen in eine Dispersion. Anstelle der Beschichtung mit einem Lack kann natürlich der Halbleiter auch lediglich vermengt mit einem Bindemittel, beispielsweise in der Art eines Leimes - wie oben beschrieben -, auf die Einlagen aufgestrichen oder aufgetragen sein.

Die Anordnung der mit Germanium oder mit einem anderen Halbleiter beschichteten plattenförmigen Einlage der Dose kann natürlich auch ohne Dose angewandt werden, jedoch ist die Platte dann der Gefahr der Beschädigung ausgesetzt.

Zur Erläuterung der Vorrichtung zur Durchführung des Verfahrens ist eine Zeichnung beigefügt, in der die Vorrichtung unter Verwendung einer Schale oder Dose (Petrischale) dargestellt ist. Die Anwendung des Röhrchens mit der Wirksubstanz, mit der Flüssigkeit gerührt wird, ist in ihrer Art so übersichtlich dargestellt, daß sich die Erläuterung vermittels Zeichnung erübrigt. In der beigefügten Zeichnung ist ein Querschnitt einer solchen Schale mit Einlage dargestellt. Die Schale ist vorzugsweise rund, sie kann jedoch auch eckig sein. Ihr Durchmesser sollte dem darauf zu setzenden Gefäß, Glas oder Becher in etwa entsprechen.

Die Schale oder Dose ist aus Glas oder aus einem Kunststoff. Das Bezugszeichen 1 bezeichnet die flächige Dose, das Bezugszeichen 2 den Deckel mit seinem Rand 3, der über den Rand 4 der Dose greift. Im Inneren der Dose liegt eine aus einzelnen Schichten gebildete Einlage, die in ihrem Durchmesser in etwa etwas geringer ist als der Innendurchmesser der Dose 1. Diese Einlage trägt das Bezugszeichen 5 und ist in vorliegender Ausführung gebildet aus zwei Lagen 6 und 7, die aus Pappe oder Kunststoff bestehen. Ihre freiliegenden oberen und unteren Seiten 8 und 9 sind mit einem Halbleiter beschichtet, vorzugsweise mit Germanium oder einer Germanium-Verbindung. Die den Halbleiter enthaltende Beschichtung kann ein Lack sein oder ein Bindemittel in der Art eines Klebers, in welchem der Halbleiter dispergiert ist oder in sonst einer Art. Es ist lediglich wichtig, daß der Halbleiter die Flächen 8 und 9 gut abdeckt, das heißt, eine dichte Schicht bildet und darauf fest haftet. Die beiden Lagen 6 und 7 sind über eine Zwischenlage aus einer Metallfolie 13, beispielsweise einer Aluminiumfolie, miteinander verbunden, zum Beispiel durch kleben oder einfach durch übereinander legen. Auf diese Weise ergibt sich ein "Element", welches eine Photonenstrahlung imitiert. Die mit dem Halbleiter nicht beschichteten Oberflächen zeigen negative Ladung und die mit dem Halbleiter beschichteten Oberflächen eine Positive Ladung.

In der oben beschriebenen Anordnung können die beiden Lagen der Platte nicht nur aus Pappe oder Kunststoff sein, sondern auch aus Leder oder Zelluloid, Die äußere Beschichtung ist ein Halbleiter im weitesten Sinne oder eine sonstige Substanz. Die Innenseite der Lage 6,7 ist unbeschichtet. Zwischen beiden Lagen ist die Aluminiumfolie 13 angeordnet, die auch ein Aluminiumblech oder ein anderes Metallblech sein kann in einer Stärke von etwa 1 - 2 mm. Durch diese Plattenanordnung erfolgt nachweislich eine Verstärkung der Halbleiterschwingungen, besonders der positiven Schwingungen oder Wellen in Lebensmitteln, Arzneimitteln und Naturstoffen, und kompensiert werden alle negativen Schwingungen, die durch technische oder terrestische Störfelder hervorgerufen werden, beispielsweise durch Wasseradern.

In der obigen Beschreibung ist die Anordnung der Platteneinlage 5 in einer zum Schutze vorgesehenen Petrischale beschrieben, auf die man dann ein Gefäß setzt, in dem sich eine Flüssigkeit befindet. Durch das Wort "Petrischale"soll die Flächengröße nicht beschränkt sein, vielmehr kann diese groß sein, mehrere dm², sodaß dann zum Schutz eine entsprechend größere Dose oder Schale eingesetzt wird und auch ein entsprechend größeres Gefäß auf deren Deckel gestellt werden kann.

Das Ganze wird nun mit dem Dosen- oder Schalendeckel 2 abgedeckt, wobei der Rand 3 des Deckels über den hochstehenden Rand 4 der Dose oder des Deckels greift. In der Zeichnung sind Deckel und Dosenunterteil auseinander gezogen gezeigt. In weiterer Ausstattung der Erfindung ist der Rand 4 oder 3 so hoch gewählt, daß, wenn die Einlage 5 in der Dose liegt, zwischen ihrer Oberfläche und der Deckelunterseite noch ein Raum bleibt, in dem eine verstärkende Substanz 11 eingefüllt werden kann. Wie oben beschrieben ist, ist diese verstärkende Substanz vorzugsweise eine Pflanzliche oder biologische Substanz in entsprechender Konfektionierung.

Die Dose dient der sicheren Unterbringung der Einlage 5 mit ihren Beschichtungen, schützt diese vor Beschädigungen und ermöglicht die Anhäufung verstärkender, organischer Substanzen in trockener, wässriger oder alkoholischer Aufbereitung, in Pulverform oder in natürlicher Form und der Deckel trägt natürlich das Gefäß 12 mit seinem flüssigen Inhalt, der die Photonenstrahlung aufnimmt, den darin befindlichen Wirkstoff durch seine Strahlung verstärkt und damit seine therapeutische Wirkung vorteilhaft erhöhend beeinflußt.

**Patentansprüche**

1. Verfahren zur Übertragung von Energie, insbesondere Photonenenergie, von einem Stoff auf eine Flüssigkeit, dadurch gekennzeichnet, daß der Stoff, dessen Energie übertragen wird, ein organischer oder anorganischer Stoff ist, der eingekapselt wird und so in einem Behälter bewegt wird, in welchem sich die Flüssigkeit befindet.

2. Verfahren zur Übertragung von Energie, insbesondere Photonenenergie, von einem Stoff auf eine Flüssigkeit, dadurch gekennzeichnet, daß der Stoff, dessen Energie übertragen wird, ein organischer oder anorganischer Stoff ist, der auf das plattenförmige Gebilde geschichtet wird, das in eine Dose eingekapselt wird und das mit einem Halbleiter vor der Einkapselung beschichtet wird.

3. Vorrichtung zur Durchführung des Verfahrens nach Anspruch 1 und 2, dadurch gekennzeichnet, daß der Stoff, der übertragen wird, in einem einendig geschlossenen Röhrchen enthalten ist, das in die Flüssigkeit eintaucht, oder in einer Dose oder Schale (1,2) enthalten ist, auf deren Deckel (2) das Gefäß mit der Flüssigkeit aufgesetzt ist.

4. Vorrichtung nach Anspruch 3 dadurch gekennzeichnet, daß in der Dose (1) oder Schale ein aus mehreren Schichten bestehendes plattenförmiges Gebilde enthalten ist, das auf seinen nach oben und unten weisenden Oberflächen (8,9) mit einem Halbleiter beschichtet ist und die zwischen sich eine Aluminiumfolie trägt.

5. Vorrichtung nach Anspruch 4 dadurch gekennzeichnet, daß der Halbleiter Germanium oder eine Germanium-Verbindung ist, daß beispielsweise in Form eines Lackes auf die freien Flächen (8) bzw. (9) aufgestrichen ist.

6. Vorrichtung nach Anspruch 3 - 5 dadurch gekennzeichnet, daß der Rand der Dose (4) eine solche Höhe aufweist, daß bei aufgesetztem Deckel (2) ein freier Raum zwischen der Deckelunterseite und -oberseite des plattenförmigen Gebildes bleibt, in dem Bezugssubstanzen in Form von pflanzlichen oder biologischen Substanzen in wässriger, alkoholischer, pulvriger Form oder natürlicher Form einbringbar sind.

# EUROPÄISCHER RECHERCHENBERICHT

Nummer der Anmeldung

## EP 90 10 8190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,A | FR-A-7 992 31   (TURENNE)<br>* das ganze Dokument *<br>— — — | 1,2,3 | A 61 N 1/16 |
| X,A | FR-E-4 813 5   (TURENNE)<br>* das ganze Dokument *<br>— — — | 1,2,3 | |
| A | GB-A-2 394 57   (BOYD)<br>* das ganze Dokument *<br>— — — | 1 | |
| A | GB-A-1 980 18   (BOYD)<br>* das ganze Dokument *<br>— — — | 1,2 | |
| A | EP-A-0 158 310   (TERWEN ET AL.)<br>* Figuren * * Seite 1, Zeile 8 - Seite 3, Zeile 17 @ Seite 5, Zeile 18 - Seite 10, Zeile 22 *<br>— — — | 1,2 | |
| A | DE-U-8 613 076   (DORN)<br>* das ganze Dokument *<br>— — — — — | 4 | |

| | | | RECHERCHIERTE SACHGEBIETE (Int. Cl.5) |
|---|---|---|---|
| | | | A 61 B<br>A 61 N |

Der vorliegende Recherchenbericht wurde für alle Patentansprüche erstellt

| Recherchenort | Abschlußdatum der Recherche | Prüfer |
|---|---|---|
| Den Haag | 17 Dezember 90 | CHEN A.H. |